# EUROPEAN PATENT APPLICATION

(11) **EP 3 155 963 A1**
(43) Date of publication of application: **19.04.2017**
(21) Application number: 16472002.1
(22) Date of filing: 07.10.2016
(51) Int. Cl.: A61B 5/024, A61B 5/11, A61B 5/00, G06F 19/00

(54) **METHOD AND AUTOMATED SYSTEM FOR MONITORING THE CONFITION OF THE FETUS**

(30) Priority: 14.10.2015 BG 112116
(71) Applicant: Ignatov, Peter Nikolaev, 1606 Sofia (BG)
(72) Inventor: Ignatov, Peter Nikolaev, 1606 Sofia (BG)
(74) Representative: Lekova, Tatyana Boyanova

(57) **Abstract**

The invention concerns a method and automated system for monitoring the condition of the fetus, providing an increased level of predictability of the condition of the fetus before and during birth. The method consists of determining three main indicators of the fetus heart function - basic heart rate, variability of heart rate and decelerations. These three main indicators are expressed quantitatively and separately or in combination they determine a quantitative biomarker hydrogen indicator (pH), which is transformed mathematically through calculating the average of the last two to nine results; depending on the average value of the biomarker, the cardiotocographic finding is categorized into three main groups- normal, suspect and abnormal.

The automated system for monitoring the condition of the fetus includes a device for electrocardiography connected to a computer. The system includes at least one more measuring module (1), connected locally or through the internet with a module for processing the signals (2) received through the measuring module (1) and a module for quantitative interpretation of the cardiotocographic findings (3). The measurement module (1) contains a device for indirect cardiotocography (4) equipped with a transducer (5) for registering uterine contractions as well as a transducer (6) for registering the cardiac pulsations of the fetus while the module for processing the signals (2) is local or remotely connected to a desktop computer configuration or laptop. The module for quantitative interpretation of cardiotocographic findings (3) includes a server (7) with software for quantitative interpretation of cardiotocographic findings where the module for quantitative interpretation (3) is connected bidirectionally with the analyzing module (8) and the predictive module (9) where the analyzing module (8) represents a local or web-based software interface.

## Description

### TECHNICAL FIELD

The invention concerns a method and automated system for monitoring the condition of the fetus, immediately before and during birth, with application into the field of obstetrics and gynecology.

### BACKGROUND

In the last 30 years, cardiotocography has transformed into the golden standard for monitoring a fetus during birth. The method successfully finds application in the diagnostics of conditions connected to oxygen insufficiency (hypoxia, metabolic acidosis and asphyxia), before as well as at the time of birth. It has been established that the continuous monitoring the fetus during birth leads to a decrease in the seizure symptomatic in newborns.

Some weaknesses and limitations do exist.

According to many long-term investigations (Error! Reference source not found) the continuous monitoring of the fetus does not result in a statistically significant decrease in the percentage of child mortality and cases of cerebral paralysis. At the same time there has been an increase in the conducted operative births.

A series of questions connected to the use of cardiotocography in the obstetric practice remain unsolved. There is as of yet no complete clarity on the physiological substrate of the changes in the cardiac activity of the fetus. The main problem is a lack of precisely defined qualitative and quantitative criteria for the identification of fetal distress. This leads to a large number of false positive results and therefore to unnecessary interventions.

As a whole, the standard indirect cardiotocography is characterized by very high sensitivity (percentage of cases where a given method correctly identifies the healthy individuals) but low specificity (percentage where a given method correctly identifies the sick individuals). This is why in modern obstetric practice there is an increased interest towards computer systems for the analysis and interpretation of cardiotocographic findings. The expectations are that these systems will help in the decrease or even elimination of the subjective factor which is considered the main prerequisite for increasing the specificity of the method.

There is a known system for the interpretation of cardiotocographic findings, based on direct cardiotocography (STAN, Neoventa Medical). An additional electrode is placed on the scalp of the fetus and in this way fetal electrocardiography (ECG) can be conducted. The STAN system compares, in real time, the findings of the cardiotocography with the ST interval of the fetal ECG. The possible elevation in the ST interval would suggest myocardial ischemia in the fetal heart. In the event of an ST interval elevation, the computer software would use visual and sound signals to notify.

In real clinical conditions this notification is usually delayed as the onset of myocardial ischemia is a result of long term oxygen insufficiency which has led to the depletion of compensatory mechanisms in the fetus and has reached the stage of affecting vital organs (heart, brain, adrenals).

There are also some practical limitations in the application of STAN. Due to the usage of direct cardiotocography for the application of this method, the following conditions must all be met:
- Presence of cervical dilation (>3cm)
- ruptured amniotic sac ;
- proven lack of vaginal infection;

The necessity of compliance with the stated conditions practically allows the use of the STAN method only during the active phase of first and the majority of the second stage of birth.

Another known system is "STG-Online" (Trium Analysis Online GmBH), which can be used with direct as well as indirect cardiotocography. It consists of an internet based application for the evaluation of the basic components of the cardiotocographic test- basic heart rate of the fetus, decelerations and variability, according to the criteria described in the last revision of the classification of FIGO (the International Federation of Gynecology and Obstetrics). This adds to the decrease of subjectivity in the interpretation of findings.

The main shortcoming of this system is that is does not provide a solution for improving the precision of cardiotocographic testing (does not increase specificity of method), but rather only helps in providing a more objective measurement of fetal condition according to FIGO criteria.

### TECHNICAL SUMMARY

The goal of the current invention is the creation of a method and automated system for monitoring the condition of a fetus, which will allow the general condition of the fetus to be determined before and during the birth with a high degree of accuracy and respectively to provide accurate prognosis and instructions to the team of doctors concerning the method of birth with which we can significantly increase the specificity of the method and therefore reach an accurate conclusion about the necessity of undertaking the appropriate obstetric actions before the occurrence of irreversible damage to the fetus.

This goal is achieved by the creation of a method for monitoring the condition of the fetus where the heart rate of the fetus is observed. It is characteristic for the method to define three main indicators of cardiac activity of the fetus which are expressed quantitatively where the measurement of the basic indicators in conducted two to nine times and the quantitative value of the indicators is defined as an average of these measurements. Each of these quantitatively defined indicators, separately or in combination define the quantitative biomarker. This biomarker is transformed mathematically where its value is tied with pH of the fetus and is categorized into three main groups- normal, suspect and abnormal.

It is suitable to generate recommendations for the undertaking of certain obstetric actions based on the conducted categorization.

In one variant of conducting the method, the measurement of the basic indicators is carried out six times with an interval of about 5 minutes between each of the measurements.

The goal is achieved through the creation of an automated system for monitoring the condition of the fetus, including a device for electrocardiography connected with a computer. It is characteristic for this system to include at least one measurement module, connected locally or through the internet with a module for the processing of the signals obtained from the measurement module and module for the quantitative interpretation of the cardiotocographic findings. The measurement module includes a device for indirect cardiotocography, supplied with a transducer for the registration of uterine contractions as well as a transducer for the registration of the fetal cardiac pulsations. The module for the processing of the signals is locally or remotely connected with a desktop computer configuration or laptop. The module for the quantitative interpretation of the cardiotocographic findings includes a server with software for the quantitative interpretation of the cardiotocographic findings.

The module for the quantitative interpretation is connected bidirectionally with the analyzing module and the predictive module. The analyzing module represents a local or web-based software interface.

In one variant of the automated system, the analyzing module is partly or fully integrated into the module for quantitative interpretation of cardiotocographic findings.

It is appropriate for the signal processing module to be connected to the module for quantitative interpretation of the cardiotocographic findings through LAN or through the World Wide Web (www).

The advantages of the method and automated system for monitoring the condition of the fetus are as follows:
The obtained assessment of the condition of the fetus is representative of the future period of time which takes up a period of 20-30 min as the method and system allow the storage of all the obtained information. This gives the opportunity to create a database with patient registers.

The most important advantage of the method and system considering the new invention compared to the STAN system consists of the fact that the birth recommendation is obtained at least 30 minutes before the occurrence of heart or brain damage caused by oxygen deficit. In that sense, the invention provides the opportunity to prevent these complications.

Unlike STAN, the usage of the method and system in accordance with the current invention does not require the placement of electrodes onto the frontal part of the fetus and therefore there is no risk of infection and no need to rupture the amniotic sack. It is not necessary to have cervical dilation (indirect cardiotocography is used).

Unlike CTG-Online, the system in accordance with the current invention contains modules which provide not only objective analysis of the obtained information but also the generation of recommendations for the undertaking of the necessary clinical action. Based on this, we can note a statistically significant increase in the specificity of the methodology.

### BRIEF DESCRIPTION OF DRAWINGS

Figure 1 represents the block scheme of the system in accordance with the current invention;
Figure 2 explain the system architecture of the QSL protocol of the system from figure 1;
Figure 3 represents, in a graphic way, standard deviations between prognoses and actual values of pH of the fetus according to the composition of the CTG-score;
Figure 4 is a graphic illustrating the 4 CTG-score groups according to the established standard deviation between prognoses and actual results for the pH of the fetus. All possible types of CTG-scores are represented (single-, double- and triple-component);
Figure 5 illustrates the set of instructions according to which the predictive module 9 functions.

### EXAMPLES OF EXECUTION

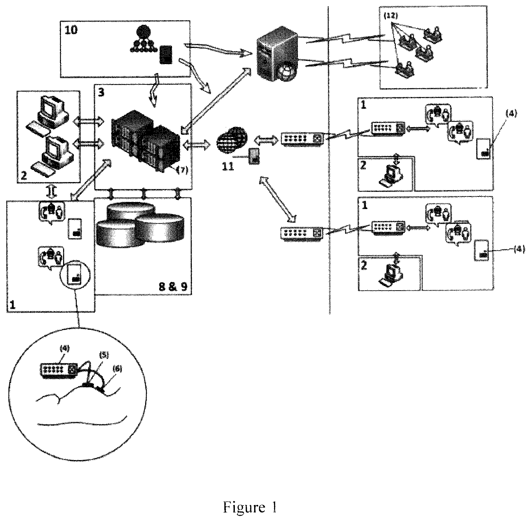

The automated system for the monitoring of the fetus (fig 1) includes at least one measuring module 1, connected locally or through the internet with a module for e processing the signals 2 received through the measuring module 1 and a module for quantitative interpretation of the cardiotocographic findings 3. The measurement module 1 contains a device for indirect cardiotocography 4 equipped with a transducer 5 for registering uterine contractions as well as a transducer 6 for registering the cardiac pulsations of the fetus. The module for processing the signals 2 is local or remotely connected to a desktop computer configuration or laptop. The module for quantitative interpretation of cardiotocographic findings 3 includes a server 7 with software for the quantitative interpretation of cardiotocographic findings and is connected bidirectionally with the analyzing module 8 and the predictive module 9 where the analyzing module 8 represents a local or web-based software interface.

The analyzing module is partly or fully integrated into the module for quantitative interpretation of cardiotocographic findings. In the event of the execution of the automated system according to the current invention, the signal processing module can be connected to the module for quantitative interpretation of the cardiotocographic findings through LAN or through the World Wide Web (www).

Module 1 includes each device capable of carrying out the mentioned functions. Examples of these kinds of devices are produced by Hewlett-Packard HP Series 50 OB TraceVue, Corometrics 170 Series of General Electric, Philips 1351A and others.

Module 2 is a local or remotely connected desktop computer configuration or laptop with the following minimal system requirements: processor Pentium Core 2 Due E 8400; 1 GB RAM memory; 10 GB hard disk; RS-232 serial port; 2D graphics accelerator; operation system Windows NT version 4. As computer components are constantly being developed and perfected, more modern configuration is recommended.

Module 3 includes a server with software for the quantitative interpretation of cardiotocographic (CTG) findings (quantitative cardiotocography- QuCTG), ex: Nexus/Gyn of Nexus AG and other systems.

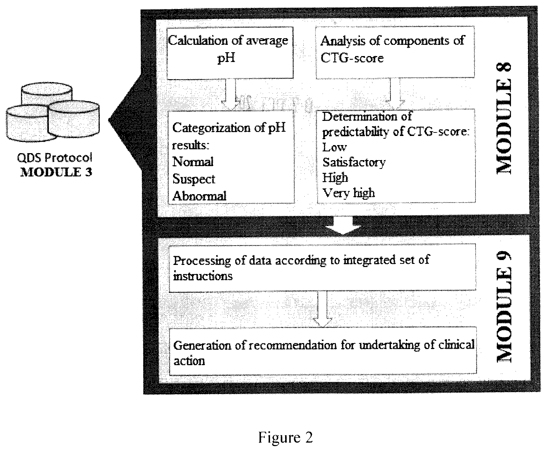

Module 3 is connected bidirectionally with modules 8 and 9. Module 8 (analysis) can represent a local or web-based software interface to which receives (from the user or completely automated) the data generated by module 3. Module 8 is connected to module 9 which based in the information processed in module 8, forms a prognosis grade of the fetal condition during the birth process based on its algorithm, shown in fig 2. The development of the system in accordance to the current invention is also possible when instead or in addition to module 2, we can use module 10 which represents a desktop computer configuration or a laptop with characteristics similar to or better to those of module 2. Module 10 can connect to module 3, 8 and 9 as well as all peripheral devices (fig1) through the World Wide Web (www) or in any other manner.

### USE OF THE INVENTION

The method provides registration of the heart pulsation of the fetus through a transducer placed on the abdominal wall of the pregnant woman. The obtained signal is then sent to specialized modules where the information is registered, processed and stored. This allows the user, for example the specialist physician, to undertake the appropriate obstetric action- for example to continue monitoring, without active involvement or to proceed to operative birth.

The data concerning uterine contraction is transmitted in the form of electrical signals through RS-232 standard of exchange of data (when **Module 1** is locally connected to **Modules 2 and** 3- fig 1) and/or LAN cable (when **Module 1** is connected to **Modules 2 and 3** through the world wide web www- fig 1). Apart from RS-232 and LAN cables, any other methods of communication can also be used.

**Module 2** transfers the obtained information to **Module 3** through a local method of exchange of information or through the World Wide Web (www). To that end, additional devices can be used such as switches, communication gateway devices, routers, server devices, providing conventional HTTPS (or other type) data exchange, voice-over-IP, protocols for video communication and others.

Module 3 (realized for example through the Nexus/Gyn system) conducts a quantitative analysis of the following three components of every CTG recording:

### Microfluctuations in the heart rate of the fetus (OSZ)

Presents the number of all following oscillation peaks below and above the basic heart rate, their oscillation amplitudes and the average value of the so called "fast (beat-to-beat) variability" for a unit of time.

### The area of all the decreases in heart rate of the fetus (DEC)

Every prolonged decrease in the heart rate of the fetus is registered (deceleration) and its area is calculated.

### Basic heart rate of fetus (FRQ)

The average frequency of the cardiac tones of the fetus is measured, rounded to an accuracy of 5 beats per minute for an interval of 10 minutes, keeping in mind that periods of highly expressed variability are ignored (>25 beats per minute).

Software module 3 for quantitative cardiotocography (QuCTG) Nexus/Gyn analyzes the obtained information, where the **micro fluctuations** of the heart rate of the fetus, **decelerations and the basic heart rate** are rated with a value from 0 to 6. On the basis of this, a grade is generated. This CTG grade represents a collection of the values (0 to 6) of the three indicators and can take on values from 0 to 18. When the values of the grade are 0, 1 and 2, it concerns a completely normal CTG recording. Values of 3, 4, 5 and 6 can also be accepted as normal but is also possible to be accompanied by suspect areas in the CTG test. All values above 6 are interpreted as pathologic and the higher the value of the grade, the higher the risk for the fetus.

There is a correlation between CTG grading and parameters of base-acid conditions (BAC) of the fetus, for example base excess (BE), base deficit (BD), buffer base (BB), partial pressure of blood gases (oxygen- pO2, carbon dioxide- pCO2) and others. The strongest correlation is established between the CTG grade and the hydrogen indicator or **"pH"** (r=-0.559, p<<10^-4), which is in the base of the mathematical model for prognosis of the pH of the fetus in utero, within the software Module 3 for quantitative cardiotocography Nexus/Gyn. According to this model, each CTG grade corresponds to a particular pH value of the pH of the fetus (table 1).

In 80% of cases, prognosis values of pH are found in the interval of -0.092 and +0.071 of the values of pH determined in the umbilical artery of the newborn immediately after birth and before the cutting of the umbilical cord.

In **Module 8** two types of information is admitted and processed, respectively, two variables. The first variable is the generated by **Module 3** predictive value of pH of the fetus. **Module 8** calculates the average value of the last few pH results (from 2 to 9). With the usage of average values, in 85% of cases, the actual values of pH of the newborn calculated from the umbilical artery immediately after birth before the cutting of the umbilical cord, are found in the interval -0.037/+0.046 of the predicted results.

According to the numerical value of the **average result of the predicted pH, Module 8** categorizes the findings into 3 categories: normal (from 7.350 to 7.237), suspect (from 7.237 to 7.137) or abnormal (<7.137) pH of the fetus.

The second variable which can be analyzed by **Module 8** is the **composition of CTG** grade. Depending on how many components take part in the formation of the grade, the following division into 3 types of CTG -grade can be expected:
- **Single component -** value of CTG grade is determined only by one component:
   ∘ Microfluctuations (OSZ)
   ∘ Or decelerations (DEC)
   ∘ Or basic heart rate of fetus (FRQ)
- **Double component** - value of the grade is formed by two components:
   ∘ Microfluctuations + decelerations (OSZ+DEC)
   ∘ Or micro fluctuations + basic heart rate (OSZ+FRQ)
   ∘ Or basic heart rate + decelerations (FRQ+DEC)
- **Triple component-** all components take part in the formation of the value of the grade.

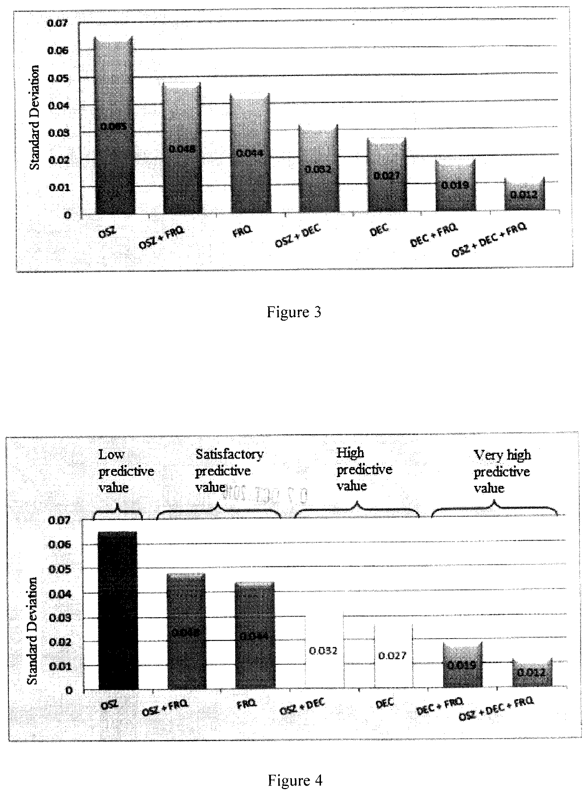

As the separate components of the CTG grade and their possible combinations have different predictive values in relation to the condition of the fetus, **Module 8** categorizes the incoming findings according to the size of the standard deviation (SD) between predicted and actual pH results (fig 3). On the basis of these criteria, 4 main grade groups are formed- with low, satisfactory, high and very high predictive value (fig 4).

**Module 8** transfers the obtained and processed information to **Module 9** (fig 2).

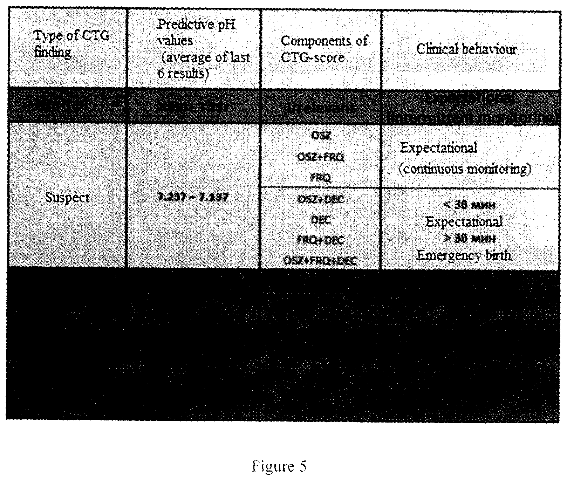

In accordance with the integrated set of instructions (fig 5), **Module 9 (Predictor)** analyzes the numerical value of the average result as predictive pH, obtained from **Module 8,** on the basis of which it is determined whether it is a **normal, suspect or abnormal CTG finding.** As a result, **Module 9** analyzes the grade based on which the **recommendation of clinical action** is generated.

**Table 1:**

| CTG-Score | pH Prognosis |
|---|---|
| | |
| 0 | 7.350 |
| 1 | 7.314 |
| 2 | 7.278 |
| 3 | 7.242 |
| 4 | 7.206 |
| 5 | 7.170 |
| 6 | 7.134 |
| 7 | 7.098 |
| 8 | 7.062 |
| 9 | 7.026 |
| 10 | 6.990 |
| 11 | 6.954 |
| 12 | 6.908 |
| 13 | 6.882 |
| 14 | 6.846 |
| 15 | 6.810 |
| 16 | 6.774 |
| 17 | 6.738 |
| 18 | 6.702 |

### 1. Normal CTG-finding

In this group fall the predictive pH results (average or other index) from **7.350** to **7.237.**

The composition of the CTG grade is not of significant importance because the predictive values of pH are high enough. In this case, according to the set of instructions, we can accept intermittent monitoring during the first and second phase of birth. (Fig 5).

### 2. Suspect CTG-finding

In this group fall the predictive pH results (average or other index) from **7.237** to **7.137.** When the CTG grade is formed by the components "micro fluctuation" (OSZ) or "micro fluctuation" + "basic heart rate" (OSZ+FRQ) or "basic heart rate" (FRQ), there is a low or satisfactory predictive value. We can expect high values of standard deviation which is found in the range of 0.044 to 0.065 (fig 4).

In the event of such high variability, there is insufficient convincing data that the actual pH values of the fetus are so decreased, which does not require a change in the clinical action. In these cases however, it is necessary to resort to additional monitoring of the fetus during the first and second phase of birth.

If the CTG grade is formed of the components "decelerations" + "micro fluctuations" (OSZ + DEC), "decelerations (DEC)", "decelerations" + "basic heart rate" (DEC+FRQ), or "decelerations" + "micro fluctuations" + "basic heart rate" (DEC+OSZ+FRQ) - there is a high or very high predictive value. We can expect significantly less standard deviation which in these cases is found in the range of 0.012 to 0.032 (Fig 4). Due to the small variability, it is expected that the predicted low pH values correlate very well with the actual values. Therefore, it is necessary to reevaluate the clinical action while in the meantime constant monitoring is assigned. If there is no improvement within 30 minutes, the recommendation is to proceed to an operative birth. (Fig 5).

### 3. Pathologic CTG- finding

In this group fall the predictive pH results (average or other index) <7.137.

If the CTG grade is formed by the components "micro fluctuation" (OSZ) or "micro fluctuation" + "basic heart rate" (OSZ+FRQ) or "basic heart rate" (FRQ), there is a low or satisfactory predictive value. We can expect high values of standard deviation which is found in the range of 0.044 to 0.065 (fig 4). This means that the actual pH of the fetus may vary considerably from the predicted value due to which there is no convincing idea about the condition of the fetus. In this case, a re-evaluation of the obstetric action is recommended due to the concerning low prognosis values of the hydrogen indicator. If there is no improvement within 30min, the recommendation is for an emergency birth.

If the CTG grade is formed of the components "decelerations" + "micro fluctuations" (OSZ + DEC), "decelerations (DEC)", "decelerations" + "basic heart rate" (DEC+FRQ), or "decelerations" + "micro fluctuations" + "basic heart rate" (DEC+OSZ+FRQ) - there is a high or very high predictive value. We can expect significantly less standard deviation which in these cases is found in the range of 0.012 to 0.032 (Fig 4). Due to the calculated extremely low predictive pH values, based on the CTG grade with high or very high predictive value, **Module 9** generates a recommendation to proceed to emergency birth as there is convincing evidence that the fetus is suffering in utero and waiting is unjustifiable. (Fig 5).

The saved information in server cluster 7 of Modules 8 and 9 and the public portal 11 can be supplied to 3^{rd} parties or legal entities 12 locally or remotely with the goal of obtaining an expert opinion, medical consultation, data storage or other activities. This can happen with the help of specialized devices (Fig 1) which use conventional HTTPS (or other type) data exchange, voice-over-IP, protocols for video communications and/or any other methods for transfer of data.

Through Module 10, activities can be carried out remotely which lead to but are not limited to service functions as general IT maintenance of the system, installation of updates, reprogramming, archiving and all other procedures necessary for the seamless functioning of the other modules.

## Claims

1. A method for the monitoring the condition of a fetus, which consists of determining three main indicators of heart function of the fetus- basic heart rate, variability of heart rate and decelerations, **characterized by** that the three main indicators are expressed quantitatively, and separately or in combination they determine a quantitative biomarker hydrogen indicator (pH), which is transformed mathematically through the calculation of the average of the last two to nine results where depending on the average value of the biomarker, the cardiotocographic finding is categorized into three main groups- normal, suspect and abnormal.

2. Method according to claim 1, where based on the analysis of the possible combinations between the three main indicators of heart rate of the fetus- basic heart rate, variability of heart rate and decelerations, an additional categorization into 4 groups is carried out- with low, satisfactory, high and very high predictive value.

3. Method according to claims 1 and 2 where based on the quantitative grade of the biomarker pH and categorization of the three main indicators of heart rate of the fetus, a recommendation is generated for the undertaking of certain obstetric behaviors.

4. Method according to claims 1, 2 and 3, **characterized by** that the measurement of the main indicators is conducted at a 5 min interval between the separate measurements.

5. Automated system for the monitoring of the condition of the fetus which includes a device for electrocardiography connected to a computer. The system includes at least one more measuring module (1), connected locally or through the internet with a module for processing the signals (2) received through the measuring module (1) and a module for quantitative interpretation of the cardiotocographic findings (3). The measurement module (1) contains a device for indirect cardiotocography (4) equipped with a transducer (5) for registering uterine contractions as well as a transducer (6) for registering the cardiac pulsations of the fetus while the module for processing the signals (2) is local or remotely connected to a desktop computer configuration or laptop. The module for the quantitative interpretation of cardiotocographic findings (3) includes a server (7) with software for the quantitative interpretation of cardiotocographic findings where the module for the quantitative interpretation (3) is connected bidirectionally with the analyzing module (8) and the predictive module (9) where the analyzing module (8) represents a local or web-based software interface.

6. Automated system according to claim 5, **characterized by** that the analyzing module (8) is partly or fully integrated into the module for the quantitative interpretation of the cardiotocographic findings (3).

7. Automated system according to claims 5 or 6, **characterized by** that the signal processing module (2) is connected to the module for quantitative interpretation of cardiotocographic findings (3) through LAN or through the World Wide Web (www).
